(19) Europäisches Patentamt
European Patent Office
Office européen des brevets

(11) EP 0 788 510 B1

(12) **EUROPÄISCHE PATENTSCHRIFT**

(45) Veröffentlichungstag und Bekanntmachung des Hinweises auf die Patenterteilung:
**26.01.2000 Patentblatt 2000/04**

(21) Anmeldenummer: **95935923.3**

(22) Anmeldetag: **10.10.1995**

(51) Int Cl.[7]: **C07K 5/065**, A61K 38/05

(86) Internationale Anmeldenummer:
**PCT/EP95/03982**

(87) Internationale Veröffentlichungsnummer:
**WO 96/11941 (25.04.1996 Gazette 1996/18)**

# (54) DIPEPTID-DERIVATE, VERFAHREN ZU IHRER HERSTELLUNG SOWIE DIESE VERBINDUNGEN ENTHALTENDE ARZNEIMITTEL

DIPEPTIDE DERIVATIVES, PROCESS FOR PRODUCING THEM AND MEDICAMENTS CONTAINING THESE COMPOUNDS

DERIVES DE DIPEPTIDE, LEURS PROCEDES DE PREPARATION ET MEDICAMENTS CONTENANT CES COMPOSES

(84) Benannte Vertragsstaaten:
**AT BE CH DE DK ES FR GB GR IE IT LI LU NL PT SE**

(30) Priorität: **14.10.1994 DE 4436772**

(43) Veröffentlichungstag der Anmeldung:
**13.08.1997 Patentblatt 1997/33**

(73) Patentinhaber: **Roche Diagnostics GmbH**
**68298 Mannheim (DE)**

(72) Erfinder:
- **KONETSCHNY-RAPP, Silvia**
  **D-68542 Heddesheim (DE)**
- **KRELL, Hans-Willi**
  **D-82377 Penzberg (DE)**
- **MARTIN, Ulrich**
  **D-68167 Mannheim (DE)**

(56) Entgegenhaltungen:
**EP-A- 0 192 135** **WO-A-93/11152**

- **TETRAHEDRON LETTERS, Bd. 36, Nr. 16, 17.April 1995 OXFORD GB, Seiten 2785-2788, M. MURAKAMI ET AL. 'Aeruginosins 98-A and B, Trypsin Inhibitors from the Blue-Green Alga Microcystis aeruginosa' in der Anmeldung erwähnt**
- **TETRAHEDRON LETTERS, Bd. 35, Nr. 19, 9.Mai 1994 OXFORD GB, Seiten 3129-3132, M. MURAKAMI ET AL. 'Aeruginosin 298-A, a Thrombin Inhibitor from the Blue-Green Alga microcystis aeruginosa' in der Anmeldung erwähnt**
- **BIOORGANIC & MEDICINAL CHEMISTRY, Bd. 3, Nr. 8, August 1995 Seiten 1019-1024, G. DE NANTEUIL ET AL. 'New Tripeptidic Thrombin Inhibitors. Influence of P2 and P3 Residues on Activity and Selectivity'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Dipeptid-Derivat. Verfahren zu seiner Herstellung und seine Verwendung in pharmazeutischen Präparaten.

Das neue Dipeptid-Derivat hat die Struktur gemäß Formel (I):

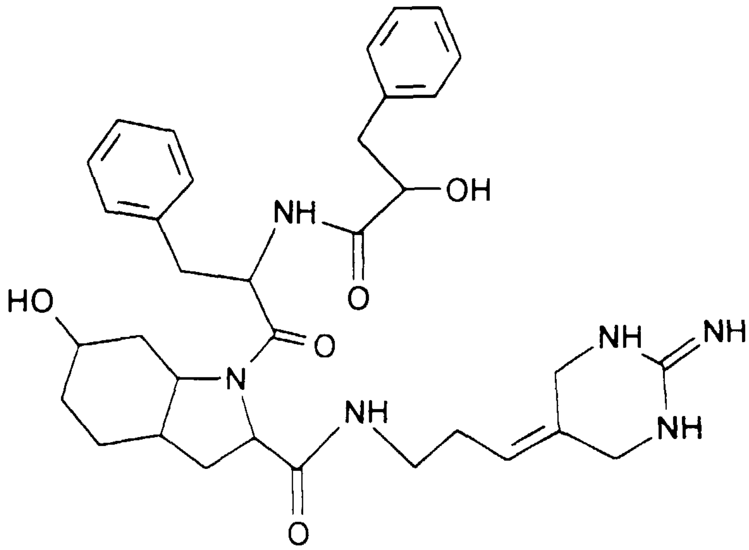

(I)

Es wird als Oscillarin bezeichnet.

Die Erfindung betrifft auch Derivate der Verbindung der Formel (I). Unter Derivaten versteht man Verbindungen der allgemeinen Formel (II) oder (III) in der R und R' gleich oder verschieden sind und Acylgruppen bedeuten, vorzugsweise die Formyl, Acetyl-, Propionyl- und Benzoylgruppe.

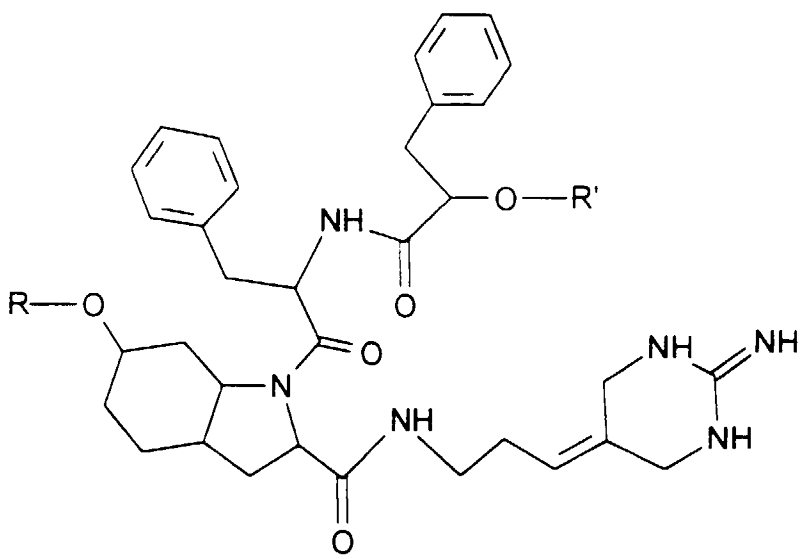

(II)

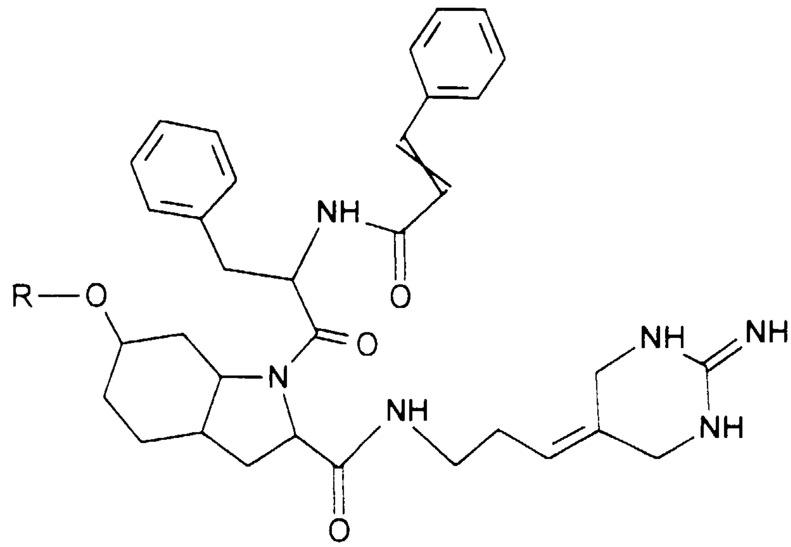

(III)

Ferner betrifft die Erfindung alle Salze der Verbindungen der allgemeinen Formeln (I) bis (III). Salze sind in erster Linie die Säureadditionssalze. Für pharmazeutische Zwecke kommen hauptsächlich physiologisch unbedenkliche Salze in Frage. Beispiele von physiologisch verwendbaren Salzen der Verbindung der Formel (I) sind Salze mit physiologisch verträglichen Mineralsäuren, wie Salzsäure, Schwefelsäure, schweflige Säure oder Phosphorsäure, oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure.

Zur Isolierung oder Reinigung können auch pharmazeutisch ungeeignete Salze Verwendung finden. Die Verbindungen der allgemeinen Formeln (I) bis (III) können solvatisiert, insbesondere hydratisiert sein. Die Hydratisierung kann im Zuge der Herstellung erfolgen oder allmählich als Folge hygroskopischer Eigenschaften der zunächst wasserfreien Verbindungen der allgemeinen Formeln (I) bis (III) auftreten.

Die Verbindungen der Formeln (I) bis (III) besitzen mehrere Asymmetriezentren. Gegenstand der Erfindung sind somit auch die optisch aktiven Formen.

Weiter wurde gefunden, daß die neuen Dipeptid-Derivate der Formeln (I) bis (III) sowie alle ihre Hydrate, Solvate und physiologisch verträglichen Salze sowohl die durch Thrombin induzierte Gerinnung von Fibrinogen im Blut als auch die durch Thrombin induzierte Aggregation der Blutplättchen hemmt. Sie verhindern damit die Entstehung von Gerinnungsthromben und von plättchenreichen Thromben und können bei der Bekämpfung und Verhütung von Krankheiten, wie Thrombosen, Apoplexie, Herzinfarkt, Entzündungen und Arteriosklerose, verwendet werden.

Thrombin, das letzte Enzym der Gerinnungskaskade, spaltet Fibrinogen zu Fibrin, das durch den Faktor XIII quervernetzt und zu einem unlöslichen Gel wird, das die Matrix für einen Thrombus bildet. Thrombin aktiviert durch Proteolyse seines Rezeptors auf den Blutplättchen die Plättchenaggregation und trägt auf diesem Weg ebenfalls zur Thrombusbildung bei. Bei der Verletzung von Blutgefäßen sind diese Prozesse notwendig, um eine Blutung zu stoppen. Unter normalen Umständen sind keine meßbaren Thrombin-Konzentrationen im Blutplasma vorhanden. Ansteigen der Thrombinkonzentration kann zur Ausbildung von Thromben und damit zu thromboembolischen Krankheiten führen, die vor allem in den Industriestaaten sehr häufig auftreten. Thrombin wird im Plasma in Form des Prothrombins bereitgehalten, und durch den Faktor Xa aus diesem freigesetzt. Thrombin aktiviert die Faktoren V, VIII und XI die dann den Faktor X in Xa umwandeln. Thrombin katalysiert dadurch seine eigene Freisetzung, weshalb es zu sehr rasch ansteigenden Thrombin-Konzentrationen kommen kann. Thrombin-Inhibitoren und Faktor Xa-Inhibitoren können deshalb die Freisetzung des Thrombins, die plättcheninduzierte und die plasmatische Blutgerinnung hemmen.

Neben Thrombin hemmen die neuen Dipeptid-Derivate der Formeln (I) bis (III) auch Trypsin, jedoch nicht Plasmin. Alle drei Enzyme gehören zur Klasse der Serinproteasen, die Peptidsubstrate neben einer basischen Aminosäure spalten.

Trypsin in ein Verdauungsenzym, das vom Pancreas bei Bedarf ausgeschüttet wird. Bei Verletzungen oder Entzündungen des Pancreas kann die dadurch bedingte erhöhte Freisetzung von Trypsin zur Gewebezerstörung führen. Trypsin-Inhibitoren können diese Gefahr eindämmen und zur Behandlung etwa der Pancreatitis eingesetzt werden.

Plasmin ist ein proteolytisches Enzym, dessen Aktivität der des Trypsins ähnelt. Es wird im Plasma unter dem Einfluß von Aktivatoren aus dem Protein Plasminogen gebildet und dient zur Auflösung der Thromben, indem es Fibrin abbaut. Hemmung des Plasmins hätte also gerade den gegenteiligen Effekt, den man mit der Hemmung des Thrombins

erzielen möchte.

Eine dem neuen Dipeptid-Derivat der Formel (I) strukturell ähnliche Verbindung (Aeruginosin 298-A), die ebenfalls Thrombin und Trypsin inhibiert, wurde aus dem Cyanobakterium *Microcystis aeruginosa* isoliert (M. Murakami et al. (1994) Tetrahedron Lett. 35, 3129-3132). Vor kurzem wurden zwei weitere Vertreter dieses Strukturtyps mit ähnlichem Wirkprofil gegenüber Serinproteasen veröffentlicht (M. Murakami et al. (1995) Tetrahedron Letters 36, 2785-2788). Ferner wurden aus *Microcystis aeruginosa* die Micropeptine A, B und 90 als potente Trypsin- und Plasmin-Inhibitoren beschrieben (T. Okino et al. (1993) Tetrahedron Lett. 34, 8131-8134; K. Ishida et al. (1995) Tetrahedron Lett. 36, 3535-3538). Von N. Fusetani et al. wurden Thrombin-Inhibitoren aus dem Meeresschwamm *Theonella sp.* publiziert: die Cyclopeptide Cyclotheonamid A und B, die auch Trypsin und Plasmin hemmen (N. Fusetani et al. (1990) J. Am. Chem. Soc. 112, 7053-7054) sowie das lineare Peptid Nazumamid A, das kein Trypsin-Inhibitor ist (N. Fusetani (1991) Tetrahedron Lett. 32, 7073-7074).

Man erhält das neue Dipeptid-Derivat der Formel (I), wenn man geeignete Blaualgen (Cyanobakterien), vorzugsweise der Art *Oscillatoriae agardhii,* in üblicher Weise in einer mineralischen Nährlösung, der Spurenelemente zugesetzt sind, unter Dauerbelichtung kultiviert und das Produkt der Formel (I) nach den üblichen Methoden isoliert.

Die Erfinding betrifft auch Kulturbrühen und die aus diesen gewonnenen Extrakte und Konzentrate, welche die Verbindung der Formel (I) enthalten.

Bei Kenntnis der Eigenschaften der neuen Verbindung der Formel (I) ist es mit Hilfe von üblichen chromatographischen, spektroskopischen und/oder Gerinnunes/Enzymtests (Thrombin, Trypsin) leicht möglich, in Routineverfahren geeignete Cyanobakterienstämme auszusuchen, welche die erfindungsgemäße Verbindung der Formel (I) produzieren.

Zur Gewinnung des Dipeptid-Derivats der Formel (I) eignen sich bevorzugt Cyanobakterien der Art *Oscillatoria agardhii* Gomont. Die Algen können von öffentlich zugänglichen Algensammlungen, wie z.B. der American Type Culture Collection (ATCC) oder der Sammlung von Algenkulturen in Göttingen, bezogen werden. Alternativ können auch Eigenisolate von *Oscillatoria agardii* verwendet werden. Sie lassen sich aus Wasserproben von meso- oder eutrophen Seen, bevorzugt in Mittel- und Nordeuropa, mit klassischen und konventionellen Methoden isolieren. Seit Jahren entwickelt sich *Oscillatoria agardhii* in holländischen hypertrophen Seen zur dominanten Spezies (C. Berger, (1975) Int. Ver. Theor. Angew. Limnol. Verh. 19, 2689-2697).

Für die Eigenisolation können die Algen mittels eines konventionellen Planktonnetzes aus dem Gewässer gefischt werden. Vorzugsweise werden die Algen direkt einer Algenblüte entnommen. Für eine Erstanreicherung eignen sich Nährböden mit einer Zusammensetzung wie im folgenden beispielhaft beschrieben:

Nährmedium 1:

0.7 g $KNO_3$, 0.05 g $Ca(NO_3)_2$ x 4 $H_2O$, 0.1 g $NaNO_3$, 0.2 g $MgSO_4$ x 7 $H_2O$, 0.4 g $K_2HPO_4$, 5 ml Spurenelementlösung, 15.0 g Agar, 1000 ml deionisiertes Wasser, pH 7.5.
Spurenelementlösung: 800 mg NaFeEDTA x 2 $H_2O$, 10 mg $MnCl_2$ x 4 $H_2O$, 2 mg $CoCl_2$, 1 mg $CuSO_4$, 1 mg $Na_2MoO_4$ x 2 $H_2O$, 2 mg $ZnCl_2$, 0.5 mg LiCl, 0.5 mg $SnCl_2$ x 2 $H_2O$, 1 mg $H_3BO_3$, 2 mg KBr, 2 mg KJ, 0.5 mg $BaCl_2$, 1000 ml deionisiertes Wasser, pH 6.

Weitere Isolierungsmethoden sind bei E.G. Pringsheim (Algenreinkulturen, VEB Gustav Fischer, Jena, 1954) zu finden. Oscillatorien haben keine Heterocysten und bilden keine Sporen. Die Trichome bewegen sich durch charakteristiche pendelartige Schwingungen. Sie besitzen keine Gallertscheide. Die scheibenförmigen Zellen enthalten immer eingewachsene Querwände unterschiedlichen Alters.

Zur Anzucht von *Oscillatoria agardhii* und somit zur Produktion von Oscillarin können alle gängigen Kulturverfahren angewendet werden, die eine ausreichende Biomassenproduktion ermöglichen. Die Bildung von Oscillarin ist prinzipiell unabhängig von den verwendeten Kulturgefäßen. Kultivierung auf Nähragar, Roller und Schüttelkolben sowie alle beschriebenen Fermentertypen eignen sich gleichermaßen. Die Anzucht erfolgt in allen Fällen unter sterilen Bedingungen. Die Beimpfung der Nährmedien erfolgt nach allgemein üblichen Methoden, z.B. Schrägröhrchen oder Kolbenkulturen.

Die Kultur kann in allen Nährmedien durchgeführt werden, welche bekannterweise zur Kultivierung von Cyanobakterien verwendet werden.

Bevorzugt wird ein mineralisches Grundmedium nach Zielinski in leicht modifizierter Form eingesetzt:

Nährmedium 2:

3.6 mg $CaCl_2$ x 2 $H_2O$, 20.3 mg $Ca(NO_3)_2$ x 4 $H_2O$, 2.7 mg KCl, 1.5 mg $K_2HPO_4$, 76.0 mg $MgSO_4$ x 7 $H_2O$, 84.0 mg $NaHCO_3$, 1 ml Spurenelementlösung, 1000 ml deionisiertes Wasser.
Spurenelementlösung: 875 mg $Na_4EDTA$, 9.7 mg $Co(NO_3)_2$ x 6 $H_2O$, 284 mg $FeCl_2$ x 6 $H_2O$, 72.2 mg $MnCl_2$ x 4 $H_2O$, 25.2 mg $Na_2MoO_4$ x 2 $H_2O$, 43.7 mg $ZnSO_4$ x 7 $H_2O$, 1000 ml deionisiertes Wasser.

(K. Zielinski, Dissertation, Univ. Freiburg, 1988, S.23)

Alternativ kann auch ein modifiziertes Medium nach A. Zehnder und E.O. Hughes verwendet werden:

Nährmedium 3:

200 mg $NaNO_3$, 10 mg $NH_4Cl$. 65 mg $K_2HPO_4$ x 3 $H_2O$, 50 mg $MgSO_4$ x 7 $H_2O$, 13 mg $CaCl_2$ x 2 $H_2O$, 1 ml Spurenelementlösung, 1000 ml deionisiertes Wasser. Spurenelementlösung: wie Nährmedium 2 (A. Zehnder, E.O. Hughes (1958) Can. J. Microbiol. 4 309-408)

Die Kulturen werden üblicherweise dauerbelichetet, z.B. mit Philips "warm white" fluorescent lamp TLE 22W/ 33 (durchschnittliche Bestrahlung z.B. 30 $Wm^{-2}$) und ständig mit sterilfiltrierter Raumluft begast. Die Wachstumstemperatur kann zwischen etwa 15 und etwa 35 °C, vorzugsweise bei etwa 20 °C liegen. Der pH-Wert der wachsenden Kulturen sollte zwischen 7 und 10, vorzugsweise auf 8.5 eingestellt sein. Ein Anstieg des pH-Werts der Nährlösung in den stärker alkalischen Bereich (durch Photosynthese) sollte vermieden werden z.B. durch Zudosieren von gasförmigem $CO_2$. Licht- und Temperaturwechsel können beliebig verändert werden, wobei die Ausbeute an Oscillarin beeinflußt wird.

Es ist zweckmäßig sicherzustellen, daß die Cyanobakterien ausreichend mit Nährstoffen in Kontakt gebracht werden. Dies kann nach den allgemein üblichen Methoden wie Rühren, Schütteln und Begasen erfolgen.

Die Dauer der Züchtung kann stark variiert werden, wobei z.B. die Zusammensetzung des Nährmediums und die Züchtungstemperatur eine Rolle spielen. In der Regel beträgt die Fermentationsdauer 8 bis 30 Tage. Die jeweiligen optimalen Bedingungen können von jedem Fachmann auf dem mikrobiologischen Gebiet leicht festgelegt werden.

Alle Abweichungen und Modifikationen von den beschriebenen Kulturbedingungen sind möglich.

Die Aufarbeitung der Kulturen erfolgt nach üblichen Methoden durch Abtrennen der Algenzellen mittels Zentrifugation oder Filtration. Zur Gewinnung des Dipeptid-Derivats der Formel (I) werden die abgetrennten Zellen mit Hilfe der üblichen Methoden z.B. durch Extraktion mit organischen Lösungsmitteln wie z.B. Methanol aufgeschlossen. Dazu kann die Zellmasse auch vorher lyophilisiert werden.

Die Verbindung der Formel (I) kann in geringeren Mengen auch im Kulturüberstand enthalten sein, aus dem sie entsprechend üblicher Methoden, wie z.B. Adsorption an geeignete Harze, Ionenaustaucher oder Aktivkohle gewonnen werden kann.

Aus den gewonnenen organischen Rohextrakten kann die Verbindung der Formel (I) mit Hilfe der üblichen Extraktions-, Adsorptions-, Fällungs- und/oder Chromatographieverfahren isoliert und gegebenenfalls feingereinigt werden. Dazu können alle geeigneten Lösungsmittel und Lösungsmittelgemische sowie alle üblichen anorganischen und organischen Trennphasen, wie z.B. unmodifizierte und modifizierte Kieselgele, Ionenaustauscher, gelchromatographische Phasen oder Adsorberharze eingesetzt werden. Bevorzugt erfolgt die Isolierung des Dipeptid-Derivats der Formel (I) nach folgendem Verfahren:

Das Lyophilisat der abzentrifügierten Zellmasse wird mit Methanol aufgeschlossen. Anschließend wird der organische Extrakt einer Flüssig/flüssig-Verteilung zwischen Wasser und Butanol unterworfen. Der Butanolextrakt wird an einer CN-modifizierten Kieselgelphase mit Hilfe eines Wasser/Acetonitril-Gradienten unter sauren Bedingungen, bevorzugt bei $pH < 3$ aufgetrennt. Die die Verbindung der Formel (I) enthaltenden Fraktionen werden anschließend an Kieselgel z.B. mit Chloroform/Methanol/Eisessig/Wasser (65:25:3:4) als Fließmittel chromatographiert.

Die weitere Aufreinigung kann mittels mehrerer hochdruckflüssigkeitschromatographischer Schritte an einer C18-modifizierten Kieselgelphase erfolgen. Als Fließmittel werden Actonitril/Wasser-Gemische bzw -Gradienten mit unterschiedlichen pH-Werten eingesetzt. Nichtflüchtige Puffer können nach den üblichen Methoden z.B. durch Reversed-Phase-Chromatographie über eine C 18-Phase oder durch Gelchromatographie abgetrennt werden.

Je nach verwendetem Puffer werden unterschiedliche Salze der Verbindung der Formel (I) erhalten, wie z. B. das Trifluoroacetat bei Verwendung von Trifluoressigsäure oder das entsprechende Phosphat bei Verwendung von Phosphatpuffern. Das Gegenanion der Verbindung der Formel (I) in diesen Salzen kann nach den allgemein üblichen Methoden, wie z.B. mit Hilfe eines Anionenaustauschers gegen ein anderes Gegenion ausgetauscht werden.

Aus ihren Lösungen kann die erfindungsgemaße Verbindung nach den üblichen Methoden, z.B. Verdampfen des Lösungsmittels, Gefriertrocknung usw. erhalten werden.

Um bei den oben angegebenen Isolierungs- und Reinigungsmethoden die Fraktionen herauszufinden, in welchen die erfindungsgemäße Verbindung in höchster Konzentration bzw. Reinheit vorliegt, können die üblichen physikalisch-chemischen Methoden, z.B. HPLC, DC oder biochemische Methoden wie z.B. Bestimmung der Thrombinzeitverlängerung oder der Hemmung der enzymatischen Aktivität von Thrombin oder Trypsin herangezogen werden.

Die Verbindungen der allgemeinen Formel (II) stellt man aus dem Dipeptid-Derivat der Formel (I) nach an sich bekannten Methoden dar. Die Umsetzung erfolgt mit Acylierungsreagenzien wie Carbonsäuren unter Wasserabspaltung oder durch aktivierte Carbonsäurederivate wie Säurechloride und -anhydride oder aktivierte Ester in einem neutralen Lösungsmittel wie Toluol, Dichlormethan, Diethylether oder Tetrahydrofüran bei Temperaturen zwischen -20°C und dem Siedepunkt des Lösungsmittels, vorzugsweise in Gegenwart von Basen wie Triethylamin, Dimethylaminopyridin oder in Pyridin, das man als Lösungsmittel einsetzt.

Verbindungen der allgemeinen Formel (III) stellt man durch Wasserabspaltung aus der Verbindung der Formel (I) und anschließender Acylierung entsprechend den oben für Verbindungen der Formel (II) genannten Methoden dar. Die Wasserabspaltung geschieht bevorzugt in einem neutralen Lösungsmittel wie Toluol, Dichlormethan, Diethylether oder Tetrahydrofüran in Gegenwart von Säuren wie Salzsäure. Schwefelsäure oder Trifluoressigsäure.

Zur Herstellung von Arzneimitteln werden die Substanzen der allgemeinen Formenl (I) bis (III) und ihre Salze mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl. z.B. in Olivenöl, suspendiert oder gelöst.

Die Substanzen der allgemeinen Formeln (I) bis (III) und ihre Salze können in flüssiger oder fester Form enteral oder parenteral appliziert werden. Als Iniektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Komplexbildner (wie Ethylendiamintetraessigsäure und deren untoxische Salze) und hochmolekulare Polymere wie flüssiges Polyethylenoxid zur Viskositätsregulierung. Feste Trägerstoffe sind z.B. Stärke, Lactose, Mannit, Methylcellulose, Talcum, hochdisperse Kieselsäuren, hochmolekulare Fettsäuren (wie Stearinsäure), tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch einmal pro Tag 1-2 Tabletten mit 20-700 mg Wirkstoff gegeben werden müssen. Der Wirkstoff kann auch durch Injektion 1-8 mal pro Tag oder durch Dauerinfüsion gegeben werden, wobei 50-2000 mg pro Tag normalerweise ausreichen.

Herstellungsbeispiele

Beispiel 1

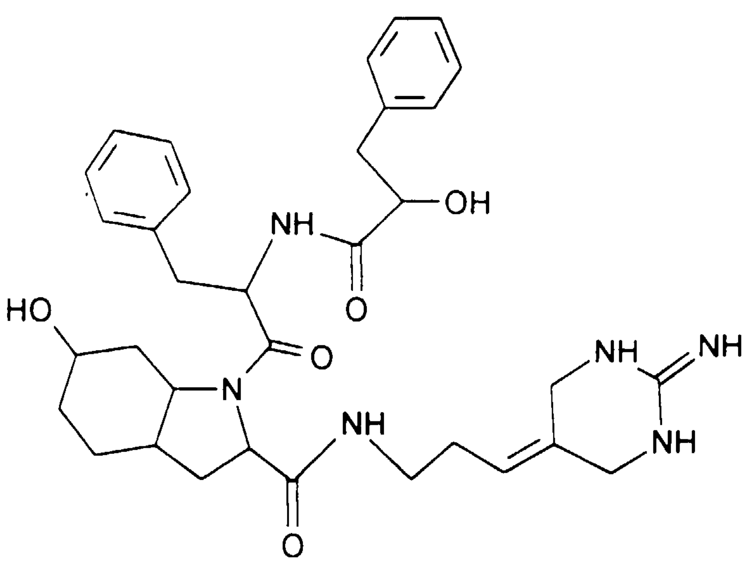

(I)

Produktion der Biomasse

Drei 20 l Glaszylinder, die mit einer pH-Regeleinheit ausgestattet sind, werden mit dem oben angegebenen, sterilfiltierten Nährmedium 2 beschickt, mit jeweils 50 ml Vorkultur von *Oscillatoria agardhii* beimpft und bei einem konstanten pH von 8.5, einer Temperatur von 20 °C und einer durchschnittlichen Bestrahlung von 30 $Wm^{-2}$ unter ständiger Begasung mit sterilfiltierter Raumluft ca. 14 Tage inkubiert

Aufarbeitung der 3x20 L Kulturen:

Zum Erntezeitpunkt werden die drei 20 l Kulturen vereinigt und die Zellen und Nährlösung durch schonende Zentrifugation (5000g, 10 min) getrennt. Die Biomasse wird bei -20°C eingefroren und anschließend lyophilisiert. Der Überstand (ca. 57 l) wird über einen starken Kationenaustauscher (Bio Rad AG 50W-X8/$H^+$-Form. 2 kg) gegeben, der anschließend mit 5 l Wasser gewaschen wird. Von diesem wird die Verbindung der Formel (I) mit 10 1l M Ameisensäure eluiert und das Eluat bei 40 °C am Rotationsverdampfer eingeengt.

Das Lyophilisat (ca. 30 g) wird einmal mit 1000 ml und zweimal mit je 500 ml Methanol extrahiert und die vereinigten Methanolphasen zur Trockne eingeengt. Der Methanolextrakt (ca. 7 g) wird in in 500 ml Wasser aufgenommen und dreimal mit je 500 ml Butanol ausgeschuttelt. Die Butanolphasen werden vereinigt und bei 40 °C am Rotationsverdampfer zur Trockne konzentriert. Analog wird mit dem konzentrierten Eluat der Kationenaustauschersäule (ca. 30 g) verfahren.

Die Butanolextrakte (ca. 5 g aus dem Lyophilisat bzw. ca. 2 g aus dem Kationenaustauschereluat) werden vereinigt, in 150 ml Methanol aufgenommen und auf ca. 25 g LiChroprep-CN-Phase (25-40 µm) aufgezogen und über eine LiChroprep-CN-Säule chromatographiert (Säule: 52 x 356 mm; Fließmittel-Gradient: Wasser/Acetonitril/Trifluoressigsäure von (100:0:0.1) bis (50:50:0.1), Gesamtelutionsvolumen: 5 l).

Die die Verbindung der Formel (I) enthaltenden Fraktionen die z.B. mit Hilfe der DC oder des Thrombinzeitverlängerungsassays identiziert werden können, werden vereinigt und bei 40 °C am Rotationsverdampfer zur Trockne eingeengt.

Das Konzentrat (ca. 1 g) wird in 100 ml Methanol gelöst, auf ca. 5 g Kieselgel LiChroprep Si60 (15-25 µm) aufgezogen und über eine Kieselgelsäule (26 x 360 mm) mit 1500 ml Chloroform/Methanol/Eisessig/Wasser (65:25: 3:4) als Fließmittel chromatographiert.

Die vereinigten (I) enthaltenden und aufkonzentrierten Fraktionen (ca. 200 mg) werden zur weiteren Reinigung in 3 ml Methanol aufgenommen und über eine Hochdruckfküssiekeitschromatographie an Nucieosil-100 RP18 (10 µm. Säule: 20 x 250 mm) mit Wasser/Acetonitril/Trifluoressigsäure (70:30:0.1) als Fließmittel weiter getrennt.

Die aufkonzentrierte; die Verbindung der Formel (I) enthaltende Fraktion (20 mg) wird in 1 ml Methanol aufgenommen und einer weiteren Hochdruckflüssigkeitschromatographie unterworfen (Säule: Nucleosil-100 RP18, 10 µm, 20 x 250 mm; Fließmittel: 50 mM Phosphatpuffer pH 7.8/Acetonitril (70:30)). Die nur (I) enthaltende Fraktion wird auf ca. 5 ml wässrige Lösung eingeengt.

Zur Entfernung des Puffers wird das Konzentrat über eine Nucleosil-100 RP18-Säule (15 x 100 mm) gegeben, wobei die Verbindung der Formel (I) gebunden wird. Nach Waschen mit 100 ml Wasser erfolgt die Elution von (I) mittels 200 ml Wasser/Methanol (10:90). Das methanolische Eluat, das nur die Verbindung der Formel (I) enthält, wird am Rotationsverdampfer bei 40°C zur Trockne eingeengt. Man erhält ca. 3 mg reines (I) als Phosphatsalz.

Analysendaten von (I), welche auch zur Charakterisierung der Verbindung geeignet sind:

**$^1$H-NMR (500.14 MHz) [a]:**

δ 7.87 (t, 1H), 7.33-7.17 (Aromaten, 10 H), 5.64 (verbreitert, 1H), 4.8 (1H), 4.25 1dd, 3.8, 7.2 Hz, 1H), 4.18 (unres. m, 4H), 4.13 (t, 9.3 Hz, 1H), 4.00 (unres. m, 1H), 3.64 (m, 1H), 3.42 (m, 1H), 3.28 (m, 1H), 3.07 (dd, 3.4,13.9 Hz, 1H), 2.89 (dd, 5.7, 12.7 Hz, 1 H), 2.83 (dd, 7.7, 13.9 Hz, 1H), 2.81 (dd, 9.4, 12.7 Hz, 1H), 2.41 (m, 2H), 2.13 (m, 1H), 1.97 (dt, 12.5, 7.5 Hz, 1H), 1.82 (m, 1H), 1.77 (dt, 10.2, 12.5, 1H), 1.58 (dt, 2.8, appr. 13 Hz, 1H), 1.43 (m, 1H), 1.42 (m, 1H), 1.41 (m, 1H), 1.33 (m, 1H).

**$^{13}$C-NMR(125.77 MHz) [a]:**

δ 174.30 (s), 171.64 (s), 139.00 (s), 137.92 (s), 137.20 (s), 130.86 (d), 130.70 (d), 129.81 (d), 129.27 (d), 128.35 (d), 127.60 (d), 120.77 (d), 73.84 (d), 66.52 (d), 61.69 (d), 56.62 (t), 56.38 (d), 55.31 (t), 53.52 (d), 41.77 (t), 40.00 (t), 38.47 (t), 37.58 (d), 34.08 (t), 31.86 (t), 29.46 (t), 26.78 (t), 19.87 (t). **LSIMS (Hochauflösung):**

617.34587 Da $[M+H]^+$

Summenformel $C_{34}H_{45}N_6O_5$

**$R_f$-Werte:**

0.66 (Butanol/Eisessig/Wasser 4:2:2, Kieselgel 60 $F_{254}$)

0.57 (Chloroform/Methanol/Eisessig/Wasser 65:25:3:4, Kieselgel 60 $F_{254}$)

**HPLC-Kapazitätsfaktor [b]:**

k' = 4.25

(Nucleosil-100 RP18: Fließmittel: Wasser/Acetonitril/Trifluoressigsäure (70:30:0.1))

[a] Bruker AMX 500 Kernresonanzspektrometer; Konzentration der Probe 13 mM Phosphatsalz in $CD_3OD$ und in $CD_3OH$; 305 K; Referenz TMS

[b] $k' = (t_R - t_m) / t_m$; $t_R$ = Gesamtretentionszeit, $t_m$ = Totzeit

## Pharmakologische Versuchsbeschreibung

### Thrombinzeit

Ein in der klinischen Gerinnungsdiagnostik gebräuchlicher Test ist die Thrombinzeit. Dieser Parameter erfaßt die Thrombinwirkung auf Fibrinogen und die Gerinnselbildung. Inhibitoren von Thrombin bewirken eine Verlägerung der Thrombinzeit.

Zur Plasmagewinnung wurden 9 Teile frisches Blut gesunder Spender mit einem Teil Natriumcitratlösung (0.11 Mol/l) gemischt und bei ca. 3000 U/min 10 Min. bei Raumtemperatur zentrifugiert. Das Plasma wurde abpipettiert und kann bei Raumtemperatur ca. 8 h aufbewahrt werden.

200 µl Citratplasma wurden in einem Kugelkoagulometer (KC10 der Firma Amelung) 2 Min. bei 37° C inkubiert. Zu 190 µl vortemperiertem Thrombin-Reagenz (Boehringer Mannheim, GmbH; enthält ca. 3 U/ml Pferdethrombin und 0.0125 M $Ca^{2+}$) gab man 10 µl Dimethylsulfoxid (DMSO) oder eine Lösung der Wirksubstanz in DMSO. Mit Zugabe dieser 200 µl Lösung zum Plasma wurde eine Stoppuhr gestartet und der Zeitpunkt bis zum Eintritt der Gerinnung bestimmt. Die Thrombinzeit betrug bei den Kontrollmessungen ca. 24 Sek. und wurde durch die Wirksubstanz der Formel (I) konzentrationsabhängig verlängert (Testkonzentration/Thrombinzeitverlängerung: 340 nM / > 300* sec; 34 nM / 65 sec; 3.5 nM / 5 sec).
*Nach 5 Min. wurde der Versuch abgebrochen.

### Thrombin-Inhibierung

Die kinetischen Messungen wurden in 0.1 M Phosphatpuffer, der 0.2 M Kochsalz und 0.5% Polyethylenglycol 6000 enthielt, bei einem pH = 75 und 25° C mit dem Substrat H-(D)-Phe-Pro-Arg-pNA (S-2238 Kabi) und humanem α-Thrombin (Sigma. spezifische Aktivität = 2150 NIH-units/mg) in Polystyrol-Halbmikrokuvetten in einem Gesamtvolumen von 1 ml durchgeführt.

In einem Vorversuch wurde bestimmt, ob die Verbindung der Formel (I) Thrombin schnell oder langsam inhibiert. Dazu wurde die Reaktion einmal durch Zugabe von 0.03 NIH-units Thrombin zu einer 100 µM-Lösung des Substrats und des Wirkstoffs gestartet. In einem zweiten Versuch wurde Substrat zu einer 5 Min. inkubierten Lösung des Thrombins und des Wirkstoffs gegeben. Die Zunahme der Konzentration von p-Nitroanilid mit der Zeit wurde spektrophotometrisch (UV-VIS-Spektrophotometer Lambda-2 der Firma Perkin-Elmer) bei 405 nm 12 Min. verfolgt. Da die bei beiden Versuchen erhaltenen Messkurven linear und parallel waren, handelt es sich bei der Wirksubstanz der Formel (I) um einen schnellen Thrombin-Inhibitor Die Inhibitionskonstante $K_i$ wurde wie folgt bestimmt. Das Substrat wurde in den Konzentrationen 100 µM. 50 µM, 30 µM, 20 µM eingesetzt und bei jeder Substratkonzentration eine Messung ohne Inhibitor und drei Messungen in Gegenwart unterschiedlicher Konzentrationen des Inhibitors der Formel (I) durchgeführt. Die Reaktionen wurden durch Zugabe von Thrombin gestartet. Die Zunahme der Extinktion bei 405 nm durch das entstehende p-Nitroanilid über einen Zeitraum von 12 Min verfolgt. Im Abstand von 20 Sek. wurden Meßpunkte (Zeit vs. Extinktion) auf einen PC übertragen. Aus den Daten wurden die Geschwindigkeiten $V_o$ (Extinktionsänderungen pro Sek.; Messungen ohne Inhibitor) und $V_i$ (Messungen mit Inhibitor) durch lineare Regression bestimmt. Benutzt wurde nur der Teil jeder Messung, bei dem sich die Substratkonzentration um weniger als 15 % vermindert hatte. Aus einer Meßreihe( konstante Inhibitorkonzentration, variable Substratkonzentrationen) bestimmte man $K_m'$ und $V_{max}$ durch nichtlinearen Fit auf die Gleichung

$$V = \frac{V_{max} * [S]}{[S] + K_m'}$$

Aus den gesamten Meßreihen berechnete man schließlich $K_i$ durch nichtlinearen Fit auf die Gleichung

$$V = \frac{V_{max} * [S]}{K_m * (1 + [S]/K_i) + [S]}$$

Die Michaeliskonstande $K_m$ betrug in allen Messungen 3.8 ± 2µM.
Die Inhibitionskonstante $K_i$ der Wirksubstanz der Formel (I) beträgt 150 nM.

### Inhibierung von Trypsin und Plasmin

10 mg Bovines pancreatisches Trypsin (Sigma) wurden in 100 ml mM Salzsaure gelöst und im Kühlschrank

aufbewahrt. 20 µl davon wurden mit 980 µl 1 mM Salzsäure versetzt. 25 µl davon wurden für jede Messung verwendet. Die Messung wurde wie für Thrombin beschrieben durchgeführt. $K_m$ = 45 µM. Die erfindungsgemäße Verbindung (I) hemmt Trypsin mit einer Inhibitionskonstante $K_i$ von 100 nM. Die Messungen mit humanem Plasmin (Sigma. 10 Units) wurden mit dem Substrat S-2251 (H-(D)-Val-Leu-Lys-pNA, Kabi) wie für Thrombin beschrieben durchgeführt. Pro Messung wurden 0.01 Units Plasmin verwendet. $K_m$ = 250 µM. Die Verbindung der Formel (I) hemmt Plasmin nicht ($K_i$ > 400 µM).

**Patentansprüche**

1. Verbindungen der Formel I

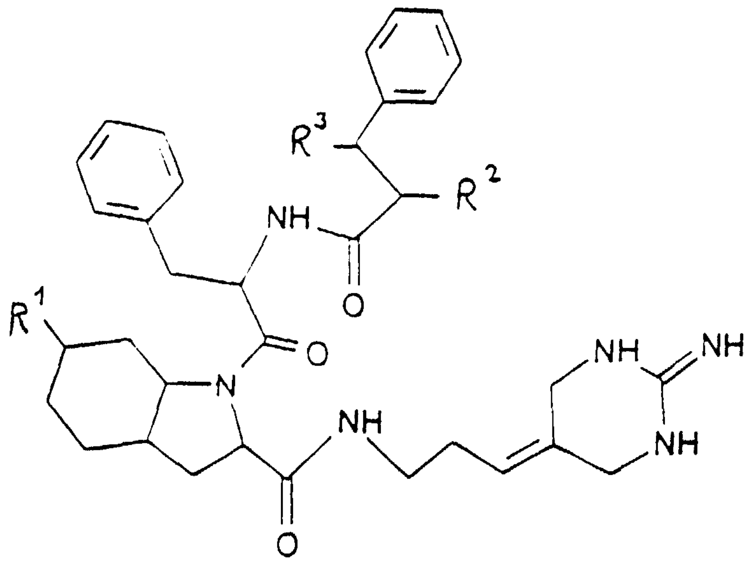

(I)

in der $R^1$ und $R^2$, die gleich oder verschieden sein können, eine Hydroxy- oder eine Acyloxygruppe bedeuten und $R^3$ Wasserstoff darstellt, wobei $R^2$ und $R^3$ gemeinsam auch einen Valenzstrich bedeuten können, deren optische aktive Formen, sowie deren pharmakologisch unbedenkliche Säureadditionssalze.

2. Verfahren zur Herstellung von Verbindungen der Formel I gemäß Anspruch 1, dadurch gekennzeichnet, daß man Cyanobakterien der Art Oscillatoria agardhii in üblicher Weise unter Dauerbelichtung kultiviert und das Produkt der Formel I, in der $R^1$ und $R^2$ Hydroxy und $R^3$ Wasserstoff bedeuten, nach üblichen Methoden isoliert und anschließend die erhaltenen Verbindungen gewunschtenfalls acyliert oder in eine andere Verbindung der Formel I überführt.

3. Verbindungen, erhältlich nach Verfahren gemäß Anspruch 2.

4. Arzneimittel, enthaltend eine Verbindung gemäß Anspruch 1 oder 3 neben üblichen Träger- und Hilfsstotfen.

5. Verwendung von Verbindungen gemäß Anspruch 1 oder 3 zur Herstellung von Arzneimitteln mit Thrombin-inhibierender Wirkung.

6. Verwendung von Verbindungen gemäß Anspruch 1 oder 3 zur Herstellung von Arzneimitteln mit Trypsin-inhibierender Wirkung.

**Claims**

1. Compounds of formula I

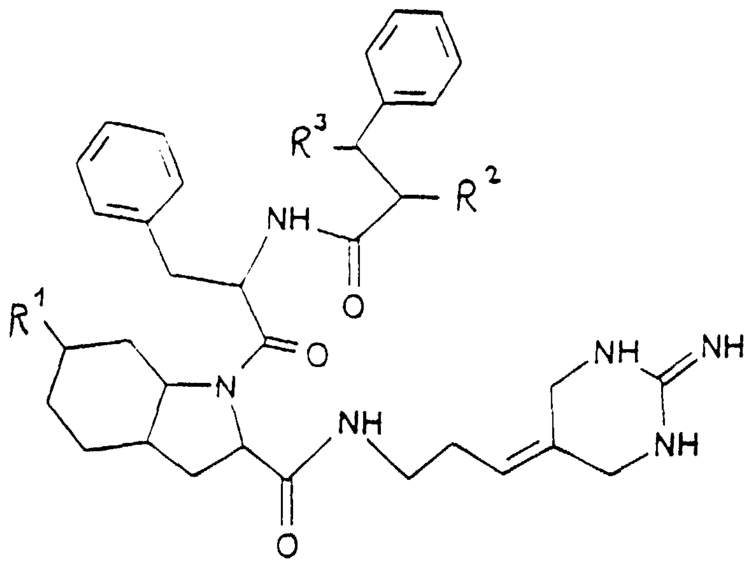

(I)

in which $R^1$ and $R^2$ can be the same or different and denote a hydroxy or an acyloxy group and $R^3$ represents hydrogen wherein $R^2$ and $R^3$ can together denote a valency dash, their optically active forms as well as pharmacologically acceptable acid addition salts thereof.

**2.** Process for the production of compounds of formula (I) as claimed in claim 1, wherein cyanobacteria of the species Oscillatoria agardhii are cultured in the usual manner under continuous light exposure and the product of formula (I) in which $R^1$ and $R^2$ denote hydroxy and $R^3$ denotes hydrogen is isolated by usual methods and if desired the compounds obtained are subsequently acylated or converted into another compound of formula (I).

**3.** Compounds obtainable by a process as claimed in claim 2.

**4.** Pharmaceutical agent containing a compound as claimed in claim 1 or 3 in addition to the usual carrier and auxiliary agents.

**5.** Use of compounds as claimed in claim 1 or 3 for the production of pharmaceutical agents with an inhibitory action on thrombin.

**6.** Use of compounds as claimed in claim 1 or 3 for the production of pharmaceutical agents with an inhibitory action on trypsin.

## Revendications

**1.** Composés de formule I

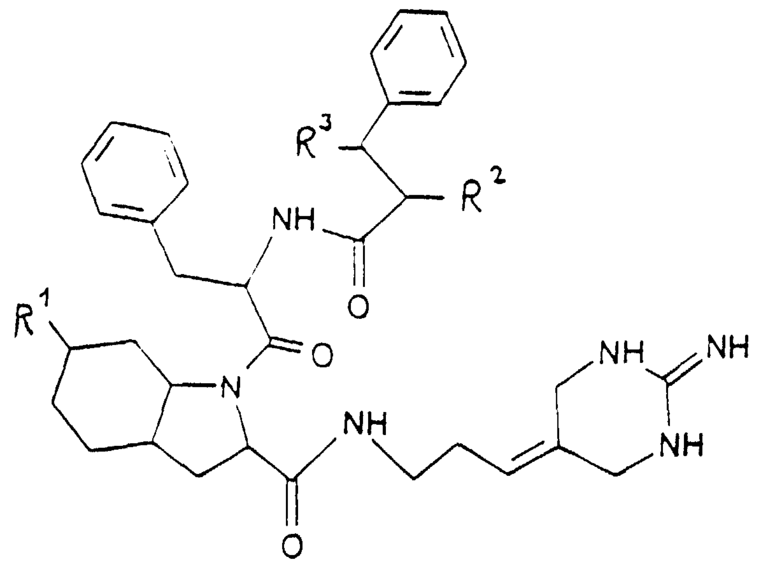

(I)

dans laquelle $R^1$ et $R^2$, qui peuvent être identiques ou différents, représentent un groupe hydroxy ou acyloxy et $R^3$ représente un atome d'hydrogène, $R^2$ et $R^3$ pouvant également représenter ensemble une valence libre, leurs formes optiquement actives et leur sels d'addition d'acide pharmacologiquement acceptables.

2. Procédé de préparation des composés de formule I selon la revendication 1, caractérisé par le fait que l'on cultive des cyanobactéries de l'espèce Oscillatoria agardhii de manière usuelle, sous éclairage permanent et selon des méthodes usuelles, on isole le produit de formule I, dans laquelle $R^1$ et $R^2$ représentent un groupe hydroxy et $R^3$ un atome d'hydrogène, et ensuite, on acyle éventuellement le composé obtenu ou on le transforme en un autre composé de formule I.

3. Composés que l'on peut obtenir selon le procédé de la revendication 2.

4. Médicament contenant un composé selon la revendication 1 ou 3, en plus de véhicules et adjuvants usuelles.

5. Utilisation de composés selon la revendication 1 ou 3, pour la préparation de médicaments ayant une activité inhibitrice de la thrombine.

6. Utilisation de composés selon la revendication 1 ou 3, pour la préparation de médicaments ayant une activité inhibitrice de la trypsine.